# EUROPEAN PATENT SPECIFICATION

(11) **EP 4 223 337 B1**
(45) Date of publication and mention of the grant of the patent: **29.01.2025**
(21) Application number: 21885310.9
(22) Date of filing: 29.10.2021
(51) Int. Cl.: A61M 16/00, A61M 16/06, A61M 16/08

(54) **NASAL PLUG FOR NASAL OXYGEN CATHETER, NASAL OXYGEN CATHETER AND VENTILATION THERAPY DEVICE**
NASENSTECKER FÜR NASALEN SAUERSTOFFKATHETER, NASALER SAUERSTOFFKATHETER UND BEATMUNGSTHERAPIEVORRICHTUNG
BOUCHON NASAL POUR CATHÉTER À OXYGÈNE NASAL, CATHÉTER À OXYGÈNE NASAL ET DISPOSITIF DE THÉRAPIE DE VENTILATION

(30) Priority: 30.10.2020 CN 202022479479 U
(43) Date of publication of application: 09.08.2023
(73) Proprietor: BMC Medical Co., Ltd., Shijingshan Beijing 100041 (CN)
(72) Inventor: CHEN, Yunjing, Tianjin 301700 (CN); LIU, Jiuzhong, Tianjin 301700 (CN); ZHENG, Fang, Tianjin 301700 (CN); ZHUANG, Zhi, Beijing 100041 (CN)
(74) Representative: Grünecker Patent- und Rechtsanwälte PartG mbB
(86) International application number: PCT/CN2021/127436
(87) International publication number: WO 2022/089584

(56) References cited:
- WO-A1-2014/089506
- WO-A1-2015/121815
- CN-A- 103 611 209
- CN-A- 110 833 647
- CN-A- 112 426 599
- CN-U- 202 961 442
- CN-U- 206 152 054
- CN-U- 209 464 462
- CN-U- 214 050 088
- US-A1- 2002 055 685
- US-A1- 2010 192 957
- US-A1- 2012 247 468
- US-A1- 2014 158 127
- US-B1- 10 314 999

## Description

### FIELD

The present disclosure relates to the field of ventilation therapy, in particular to a nasal plug for a nasal oxygen catheter, a nasal oxygen catheter including the nasal plug, and a ventilation therapy device including the nasal oxygen catheter.

### BACKGROUND

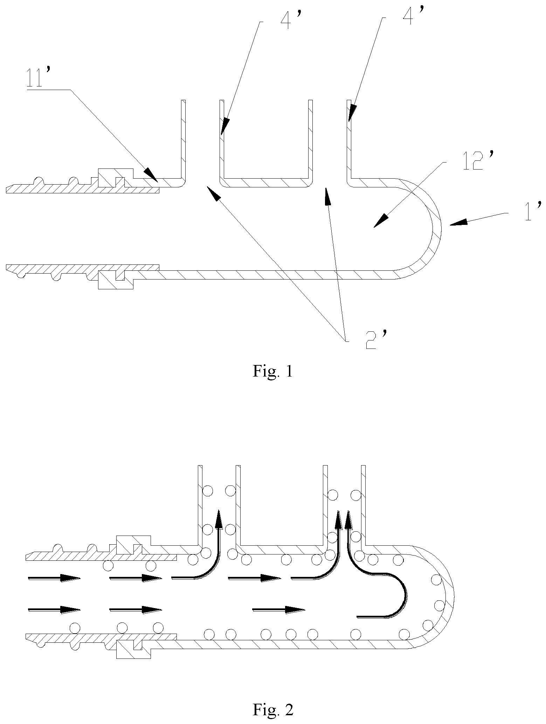

A nasal oxygen catheter is a component used in a ventilation therapy device (e.g., ventilator or high-flow oxygen therapy apparatus) to introduce pressurized air or breathable gas into the nasal cavity of a user. A nasal oxygen catheter usually has a nasal plug for insertion into the nasal cavity. Please see Fig. 1, which shows a partial structural schematic diagram of a nasal oxygen catheter according to the prior art, and highlights the structure of the nasal plug part. The nasal plug generally comprises a body 1', which comprises a casing 11', wherein a cavity 12' is formed in the casing 11' for gas flow to pass through, and the casing 11' is provided with a gas outlet 2' and also has a gas outlet tube 4' extending from the edge of the gas outlet 2' to the casing 11'. For the above-mentioned nasal plug in the prior art, the transition from the inner wall of the casing 11' to the inner wall of the gas outlet tube 4' in the cavity 12' is direct smooth transition, which leads to a problem that the condensed water in the nasal plug may enter the patient's respiratory tract through the gas outlet 2', resulting in a risk of water choking.

With reference to Fig. 2, it can be better understood that during the use of an existing ventilatory therapy device, such as a ventilator or a high-flow oxygen therapy device, there is a small amount of condensed water in the heating pipeline and nasal oxygen catheter, and the condensed water (water droplets of the condensed water are indicated by circles) flows from the heating pipeline and the body of the nasal oxygen catheter to the nasal plug of the nasal oxygen catheter in the gas flow direction (the arrow in Fig. 2 indicates the gas flow direction), and then adheres to the inner surface of the cavity 12'. The connection between the gas outlet tube 4' and the casing 11' usually has smooth transition, and the condensed water can easily enter the gas outlet tube 4' along the inner surface of the casing 11' through the smooth transition part, and then enter the respiratory tract of the patient, resulting in water choking and discomfort of the patient.

WO2015/121815 A1 discloses a respiratory nosepiece comprising a first nasal prong with a first channel extending there through, and a first side port connectable to tubes having various first and second diameters corresponding to the diameters of a first section and a second section respectively, wherein a second channel extends there through and in communication with the first channel; and wherein the second channel includes a second step between a second section and an end of the second channel.

US2012/0247468 A1 discloses a low flow heated/humidified respiratory gas delivery system, comprising a nose cannula, which may be formed with a partition that direct the flow of the respiratory gas in a first flow path of movement directly into contact with sensor through which the temperature of the respiratory gas is controlled. The respiratory gas thereafter passes in a second flow path essentially directly to the cannula outlets for application to the patient.

### SUMMARY

An object of the present disclosure is to provide a nasal plug for a nasal oxygen catheter, a nasal oxygen catheter including the nasal plug, and a ventilation therapy device including the nasal oxygen catheter, so as to solve the problem that the condensed water in the nasal plug in an existing nasal oxygen catheter is discharged from the nasal plug through the gas outlet and enters the respiratory tract of the patient.

In order to attain the above object, in a first aspect, the present disclosure provides a nasal plug for a nasal oxygen catheter. The nasal plug comprises a body, which comprises a casing and a cavity positioned in the casing; wherein the body is provided with at least one gas outlet; and the nasal plug further comprises a stop structure that surrounds the at least one gas outlet and extends from an inner wall of the casing toward the cavity, so that condensed water on the inner wall of the cavity has to flow over the stop structure before it can be discharged from the gas outlet.

Preferably, a reinforcing structure is formed on the stop structure.

Preferably, the reinforcing structure comprises reinforcing ribs.

Preferably, the reinforcing structure comprises a flanged edge, which is folded and extends towards the outside of the gas outlet.

Preferably, the stop structure comprises a first end connected to the casing and a second end extending into the cavity, and an inner diameter of the second end is greater than that of the gas outlet.

Preferably, the inner diameter of the second end is greater than that of the first end.

Preferably, the inner diameter of the first end is greater than that of the gas outlet.

Preferably, one part of the first end extends from an edge of the gas outlet, and another part of the first end is arranged spaced apart from the edge of the gas outlet.

Preferably, the stop structure is a stop ring.

Preferably, the nasal plug comprises at least two gas outlets, and the stop structure surrounds all the gas outlets on the nasal plug.

Preferably, one end of the cavity is formed as a gas inlet end, and the other end is formed as a closed end, and a connecting structure for connecting with a ventilation pipeline is formed on a side of the casing corresponding to the gas inlet end.

In a second aspect, the present disclosure provides a nasal oxygen catheter, which comprises a ventilation pipeline and the above-mentioned nasal plug.

In a third aspect, the present disclosure provides a ventilation therapy device, which comprises the above-mentioned nasal oxygen catheter.

The nasal plug for a nasal oxygen catheter in the present disclosure is provided with a stop structure, which surrounds the gas outlet and extends from the inner wall of the casing towards the cavity. As a result, the condensed water on the inner wall of the cavity has to flow over the stop structure before it can be discharged from the gas outlet. Thus, the stop structure plays a role of preventing the condensed water on the inner wall of the cavity from entering the gas outlet. Therefore, the condensed water on the inner wall of the casing can't enter into the gas outlet easily and thereby enter the patient's respiratory tract. In that way, a problem of water choking caused by the condensed water entering into the patient's respiratory tract from the gas outlet is solved.

### BRIEF DESCRIPTION OF THE DRAWINGS

The accompanying drawings are provided herein to facilitate further understanding on the present disclosure and constitute a part of this document. They are used in conjunction with the following embodiments to explain the present disclosure, but are not intended to constitute any limitation to the present disclosure. It needs to be noted especially that the arrows in the following figures represent the gas flow direction, and the circles represent condensed water. In the figures:
Fig. 1 is a partial sectional view of a nasal oxygen catheter in the prior art, in which a nasal plug part is mainly shown;
Fig. 2 shows the gas flow direction and the condensed water distribution in the structure shown in Fig. 1;
Fig. 3 is a partial sectional view of the nasal oxygen catheter according to a first embodiment of the present disclosure, in which a nasal plug part is mainly shown;
Fig. 4 shows the gas flow direction and the condensed water distribution in the structure shown in Fig. 3;
Fig. 5 is a partial sectional view of the nasal oxygen catheter according to a second embodiment of the present disclosure, in which a nasal plug part is mainly shown;
Fig. 6 is a schematic structural diagram of the stop structure in Fig. 5 when viewing from bottom to top;
Fig. 7 shows the gas flow direction and the condensed water distribution in the structure shown in Fig. 5;
Fig. 8 is a partial sectional view of the nasal oxygen catheter according to a third embodiment of the present disclosure, in which a nasal plug part is mainly shown;
Fig. 9 shows the gas flow direction and the condensed water distribution in the structure shown in Fig. 8;
Fig. 10 is a partial sectional view of the nasal oxygen catheter according to a fourth embodiment of the present disclosure, in which a nasal plug part is mainly shown;
Fig. 11 shows the gas flow direction and the condensed water distribution in the structure shown in Fig. 10;
Fig. 12 is a partial sectional view of the nasal oxygen catheter according to a fifth embodiment of the present disclosure, in which a nasal plug part is mainly shown;
Fig. 13 shows the gas flow direction and the condensed water distribution in the structure shown in Fig. 12;
Fig. 14 is a partial sectional view of the nasal oxygen catheter according to a sixth embodiment of the present disclosure, in which a nasal plug part is mainly shown;
Fig. 15 is a schematic structural diagram of the stop structure in Fig. 14 when viewing from bottom to top;
Fig. 16 shows the gas flow direction and the condensed water distribution in the structure shown in Fig. 14;
Fig. 17 is a partial sectional view of the nasal oxygen catheter according to a seventh embodiment of the present disclosure, in which a nasal plug part is mainly shown;
Fig. 18 is a schematic structural diagram of the stop structure in Fig. 17 when viewing from bottom to top;
Fig. 19 shows the gas flow direction and the condensed water distribution in the structure shown in Fig. 18.

### Reference Numbers

Prior art: 1' - body; 11' - casing; 12' - cavity; 2' - gas outlet; 4' - gas outlet tube;
Present application: 1 - body; 11 - casing; 12 - cavity; 2 - gas outlet; 3 - stop structure; 31 - reinforcing rib; 32 - flanged edge; 4 - gas outlet tube.

### DETAILED DESCRIPTION

It needs to be noted that the embodiments and the features in the embodiments can be combined freely, provided that there is no confliction among them.

In the present disclosure, it needs to be understood that the orientation or positional relationships indicated by the terms "away from" and "toward", etc. correspond to the orientation or positional relationships actually used herein; and "inside" and "outside" refer to the inside and outside with respect to the outline of each component. All those terms are used for the convenience of describing the present disclosure rather than specifying that the referred device or component may have a specific orientation or may be constructed or operated in a specific orientation. Therefore, those terms cannot be understood as constituting any limitation to the present disclosure.

In the present disclosure, unless otherwise specified and defined explicitly, the terms "install", "connect", "fix", etc. shall be interpreted in their general meaning. For example, the connection may be fixed connection, detachable connection, or integral connection; may be mechanical connection or electrical connection or in communication with each other; may be direct connection or indirect connection via an intermediate medium, or internal communication or interactive relation between two elements. Those having ordinary skills in the art may interpret the specific meanings of the terms in the present disclosure in their context.

The present disclosure will be detailed below in embodiments with reference to the accompanying drawings.

In a first aspect, the present disclosure provides a nasal plug for a nasal oxygen catheter. The nasal plug comprises a body 1, which comprises a casing 11 and a cavity 12 inside the casing 11, wherein the body 1 is provided with at least one gas outlet 2; and the nasal plug further comprises a stop structure 3 that surrounds the at least one gas outlet 2 and extends from the inner wall of the casing 11 toward the cavity 12.

The nasal plug for a nasal oxygen catheter in the present disclosure is provided with a stop structure 3, which surrounds the gas outlet 2 and extends from the inner wall of the casing 11 toward the cavity 12. As a result, the condensed water on the inner wall of the cavity 12 has to flow over the stop structure 3 before it can be discharged from the gas outlet 2. Thus, the stop structure 3 plays a role of preventing the condensed water on the inner wall of the cavity 12 from entering the gas outlet 2. Therefore, the condensed water on the inner wall of the casing 11 can't enter into the gas outlet 2 easily and thereby enter the patient's respiratory tract. In that way, a problem of water choking caused by the condensed water entering into the patient's respiratory tract from the gas outlet 2 is solved.

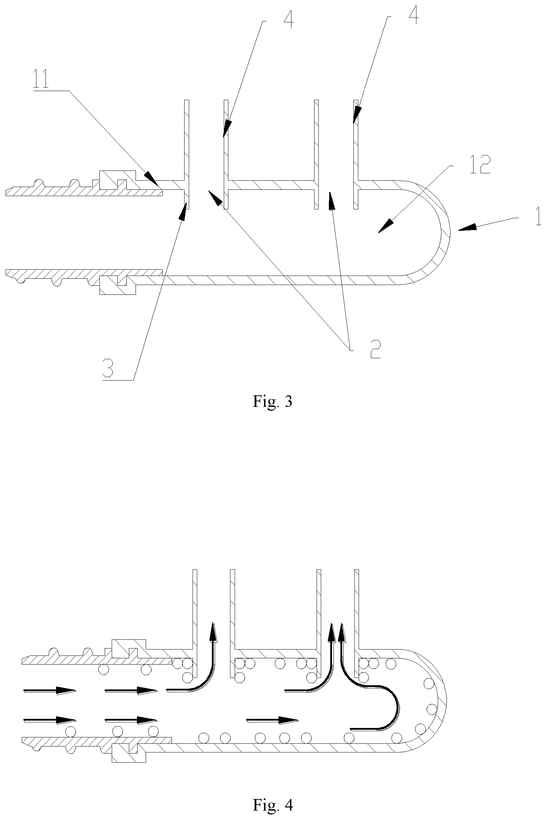

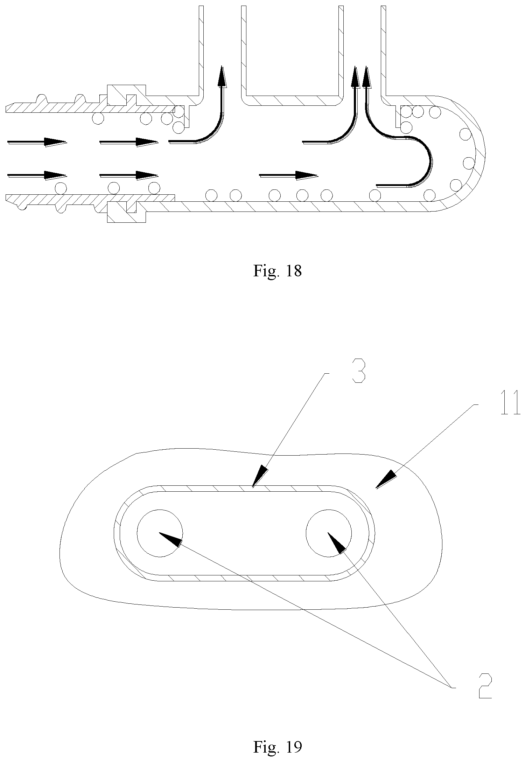

The function of the stop structure 3 in the present disclosure can be understood more easily with reference to Figs. 4, 6, 9, 11, 13, 15 and 18. During the operation of a ventilator or a high-flow oxygen therapy device, a small amount of condensed water in the heating pipeline and the nasal oxygen catheter is mainly accumulated under the inner surface of the cavity 12 in the gas flow blowing direction (under the action of gravity) after the condensed water enters the cavity 12 of the body 1 of the nasal plug. With the stop structure 3, the condensed water is also mainly accumulated on the outer circumference wall of the stop structure 3 extending into the cavity 12 (under the action of gas flow). When a relatively large amount of condensed water is accumulated on the outer circumference wall of the stop structure 3, the condensed water drips down along the outer circumference wall of the stop structure 3 to the lower inner wall of the cavity 12, so it is difficult for the condensed water to enter the gas outlet 2. Thus, the problem that the condensed water enters the respiratory tract of the patient through the gas outlet 2 is solved.

Here, the nasal plug may further be provided with a gas outlet tube 4 extending from the edge of the gas outlet 2 toward the nasal plug. The gas outlet 2 of the nasal plug is illustrated in a circular shape in the figure, and the cross section of the corresponding gas outlet tube 4 perpendicular to the axial direction is also illustrated in a circular shape. However, it can be understood that the shape of the gas outlet 2 is not limited to a circular shape, but may be an elliptical shape, a rectangular shape, or any other irregular shape, and the cross-sectional shape of the corresponding gas outlet tube 4 perpendicular to the axial direction may be changed accordingly. In addition, in the embodiments, as shown in Figs. 3 - 19, the stop structure 3 may be a stop ring, which may be arranged around one or more gas outlets 2, and the shape surrounded by the stop ring is not limited to a circular shape, but may be an elliptical shape, a rectangular shape, or any other irregular shape.

As shown in Figs. 3 and 4, in a first embodiment of the present disclosure, the nasal plug comprises two gas outlets 2, and the end of the stop structure 3 connected to the casing 11 is connected to the edges of the gas outlets 2. That is to say, the stop structure 3 extends from the edge of each gas outlet 2 into the cavity 12, and the two gas outlets 2 have their own stop structure 3, the thickness of the stop structure 3 is the same as that of the gas outlet tube 4, and the stop structure 3 may be integrated with the gas outlet tube 4. Thus, as shown in Fig. 4, owing to the existence of the stop structure 3, the edge of the gas outlet 2 is not connected directly to or directly contact with the wall of the casing 11, thereby the condensed water on the inner wall of the casing 11 can be effectively prevented from entering the gas outlet 2. In this embodiment, there may be a problem that if the material hardness of the stop structure 3 is low and the wall thickness of the stop structure 3 is thin, the stop structure 3 may be deformed and the gas outlet 2 may be blocked under a condition of high gas flow and high internal and external pressure. Therefore, preferably the problem is alleviated by increasing the material hardness of the stop structure 3, increasing the wall thickness of the stop structure 3, adding a reinforcing structure on the stop structure 3, and changing the position or shape of the stop structure 3, etc., as described in connection with other embodiments below.

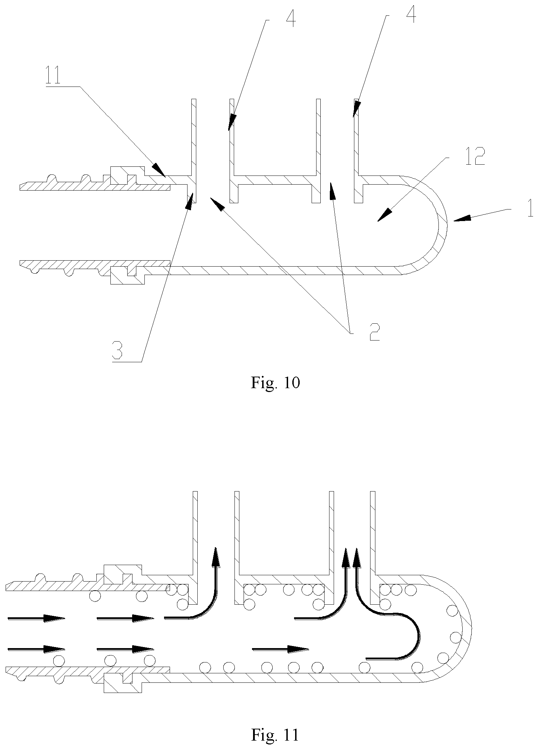

Therefore, preferably the stop structure 3 has greater wall thickness; for example, as shown in Figs. 10 and 11, compared with Figs. 3 and 4, the wall thickness of the stop structure 3 may be greater than that of the gas outlet tube 4; more specifically, the inner diameter of the stop structure 3 may be equal to that of the gas outlet tube 4, and the stop structure 3 may be integrally formed with the gas outlet tube 4. The strength of the stop structure 3 may be increased by increasing the wall thickness of the stop structure 3, thereby the problem that the stop structure 3 is deformed and the gas outlet 2 is blocked under a condition of high gas flow and high inside and outside pressure can be alleviated.

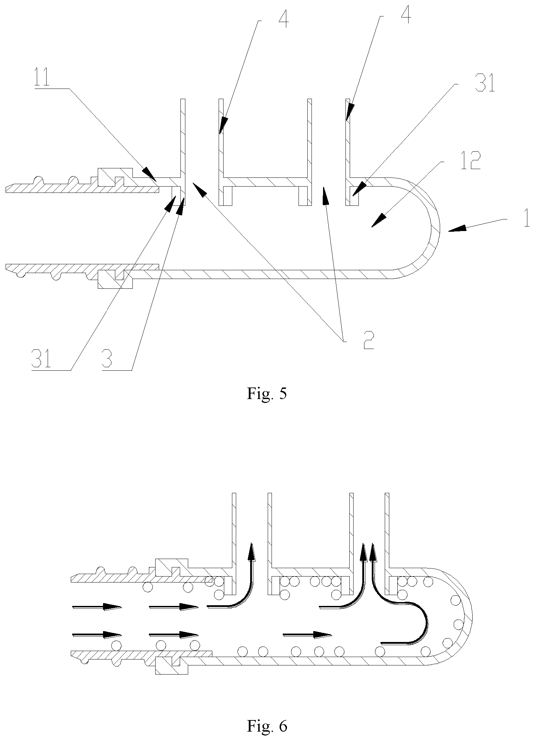

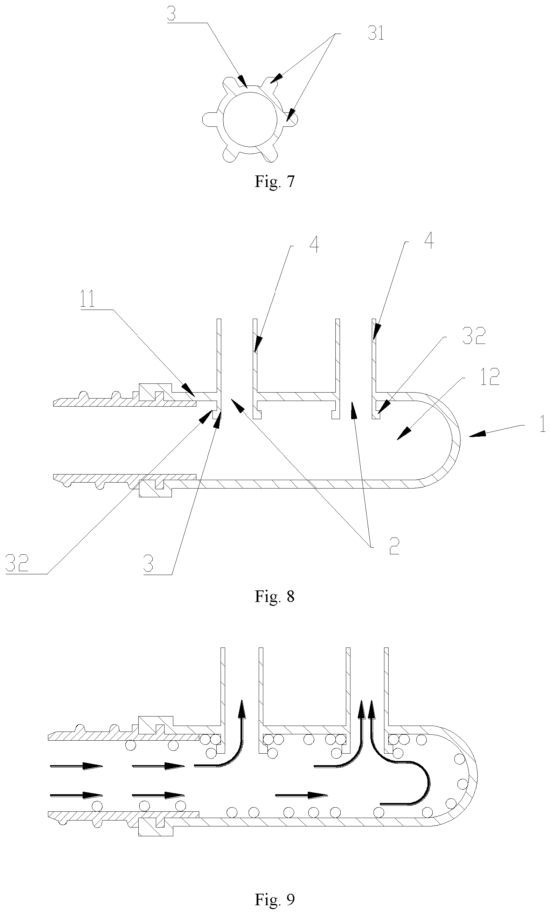

For another example, as another preferred embodiment, a reinforcing structure for improving the strength of the stop structure 3 may be formed on the stop structure 3, and the specific form of the reinforcing structure may be selected from a variety of options. For example, the reinforcing structure may comprise reinforcing ribs 31. Figs. 5 - 7 show a form of reinforcing ribs 31. In this embodiment, the stop structure 3 extends in the axial direction of the gas outlet 2, and the reinforcing ribs 31 are formed on the outer circumference wall of the stop structure 3 and extend in the axial direction of the gas outlet 2; in addition, a plurality of the reinforcing ribs 31 are formed at an interval along the outer circumferential face of the stop structure 3, grooves are formed between such reinforcing ribs 31, and condensed water may be accumulated in the grooves, thereby the condensed water on the outer circumferential face of the stop structure 3 may be guided to drip down. Of course, it can be understood that the reinforcing ribs 31 may also be arranged in any other appropriate form. For example, the reinforcing structure may be in a form of flanged edge. Figs. 8 and 9 show an arrangement of flanged edge; specifically, the end of the stop structure 3 that faces the cavity 12 has a flanged edge 32, which is folded and extends out of the gas outlet 2. In this embodiment, the flanged edge 32 extends out of the stop structure 3. Specifically, the stop structure 3 extends in the axial direction of the gas outlet 2, and the flanged edge 32 extends in a direction perpendicular to the axial direction of the stop structure 3, so that the flanged edge 32 can effectively prevent the condensed water on the outer circumferential face of the stop structure 3 from flowing into the gas outlet 2. Of course, the flanged edge 32 may also be arranged in any other appropriate form. For example, the extending direction of the flanged edge 32 may be at a different angle other than a right angle with respect to the axial direction of the stop structure 3.

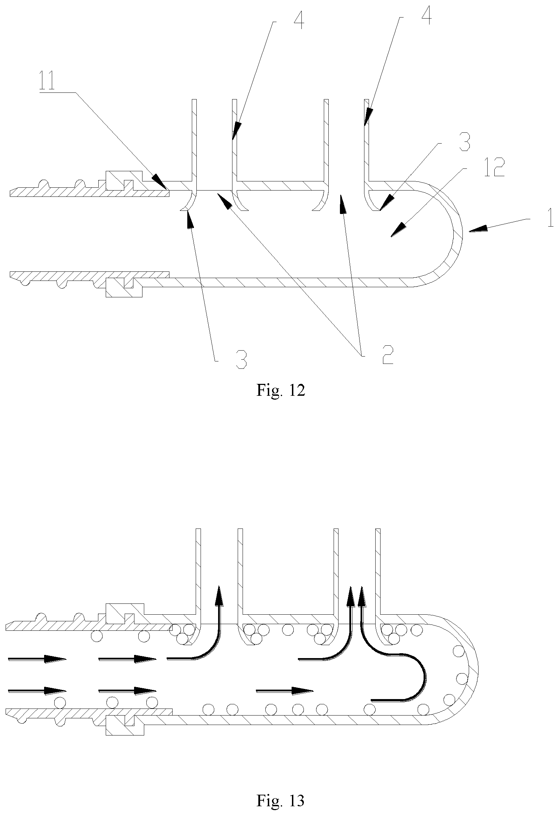

As another option, the stop structure 3 comprises a first end connected to the casing 11 and a second end extending into the cavity 12, wherein the outer diameter of the second end is greater than that of the first end; further preferably, the inner diameter of the second end is greater than that of the first end. For example, the stop structure 3 may be formed as an expanded section at least at one side adjacent to the first end, and the outer diameter of the expanded section gradually increases in the direction from the first end to the second end. In the embodiment shown in the figures, the outer diameter of the stop structure 3 gradually increases from the first end to the second end of the stop structure 3; for example, the stop structure 3 may in a bell mouth shape as shown in Figs. 12 and 13, that is to say, the entire stop structure 3 is in a bell mouth shape. In such a case, the stop structure 3 doesn't extend linearly; instead, it is in a bell mouth shape that expands from one end to the other end gradually. Thus, the strength of the stop structure 3 is increased so as to alleviate the problem that the stop structure 3 is deformed and the gas outlet 2 is blocked under the condition of high gas flow and high inside and outside pressure, and the stop structure 3 in a bell mouth shape can provide a better guiding function. In addition, it can be understood that an expanded section may be formed at the side near the first end, and the expanded section may be in the shape of a bell mouth, a tapered shape, or any other shape with the outer diameter increased gradually.

In addition, in the embodiment illustrated above, the end of the stop structure 3 connected to the casing 11 is connected to the edge of the gas outlet 2. In other words, the stop structure 3 extends from the edge of the gas outlet 2 into the cavity 12 in the casing 11; in each of the embodiments illustrated above, the nasal plug is provided with two gas outlets 2, each of which has a stop structure 3 arranged around its edge.

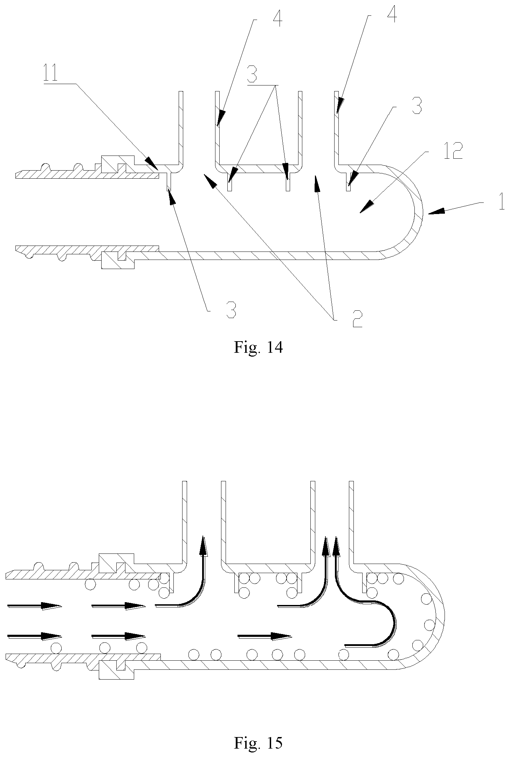

However, as another option, the stop structure 3 comprises a first end connected to the casing 11 and a second end extending into the cavity 12, wherein the inner diameter of the second end is greater than that of the gas outlet 2; for example, at least a part of the end of the stop structure 3 connected to the casing 11 is spaced from the edge of the gas outlet 2, as shown in Figs. 14, 15 and 16. For example, the end of the stop structure 3 connected to the casing 11 is fully spaced from the edge of the gas outlet 2, i.e., perpendicular to the axial direction of the gas outlet 2, and the inner diameter of the second end of the stop structure 3 is greater than that of the gas outlet 2. The cross section of the second end of the stop structure 3 is greater than that of the gas outlet 2, so that the gas flow into the gas outlet 2 from the stop structure 3 is smoother. In the case that the cross section of the second end of the stop structure 3 is greater than that of the gas outlet 2, more preferably, the inner diameter of the first end of the stop structure 3 is also greater than that of the gas outlet 2, that is to say, both the cross sections of the stop structure 3 are greater than the cross section of the gas outlet 2, the gas flow gradually enters a space with a smaller cross section at the stop structure 13 and then enters a space with a further smaller cross section at the gas outlet 2 from the gas flow passage of the nasal plug that has the maximum space, and the stop structure 13, which is a transitional region for the gas flow, can provide a gas flow guiding function to some extent. The spacing between the end of the stop structure 3 connected to the casing 11 and the edge of the gas outlet 2 is preferably slightly smaller than the length of extension of the stop structure 3 into the cavity 2; in addition, the spacing can effectively prevent the stop structure 3 from blocking the gas outlet 2.

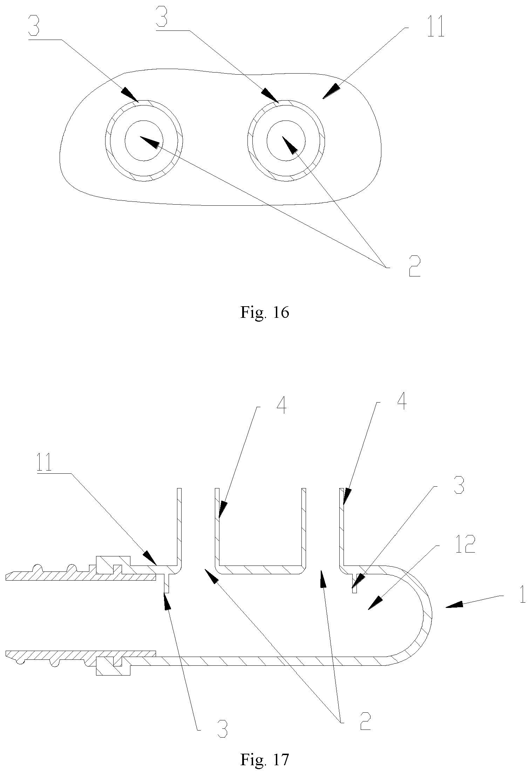

For another example, as another option, as shown in Figs. 17, 18 and 19, the nasal plug further comprises a stop structure 3 that surrounds all gas outlets 2 or at least one gas outlet 2 and extend from the inner wall of the casing 11 into the cavity 12. In the figures, the nasal plug comprises two gas outlets 2, and there is only one stop structure 3, which surrounds the two gas outlets 2, so that it is difficult for the condensed water on the inner wall of the cavity 12 outside the stop structure 3 to get over the stop structure 3 and enter the gas outlet 2. Thus, the problem of preventing the condensed water from entering the patient's respiratory tract through the gas outlets 2 is solved.

Optionally, it can be understood that at least a part of the end of the stop structure 3 connected to the casing 11 is spaced from the edges of the gas outlets 2, that is to say, only one part of the end of the stop structure 3 connected to the casing 11 may extend out of the edges of the gas outlets 2, while the other part of the end of the stop structure 3 connected to the casing 11 is arranged spaced apart from the edges of the gas outlets 2, or the other part of the end of the stop structure 3 connected to the casing 11 is fully spaced from the edges of the gas outlets 2, which may occur in the case that the stop structure 3 surrounds only one gas outlet 2 or surrounds a plurality of gas outlets 2.

It can be understood that the nasal plug usually comprises two gas outlets 2. The body 1 in the present application may be an integral and separate part, or may be formed by two or more parts assembled together, or may be formed integrally with the stop structure 3, for example, by injection-molding integrally.

In addition, as shown in the figures, one end of the cavity 12 is formed as a gas inlet end, and the other end of the cavity 12 is formed as a closed end, and a connecting structure for connecting a ventilation pipeline is formed on the side of the casing 11 corresponding to the gas inlet end; the cavity 12 may comprise a ventilation channel perpendicular to the axial direction of the gas outlet 2 (from the gas inlet end to the closed end), the gas flows into the gas inlet end and exits the gas outlet 2, the ventilation channel has a first dimension (the dimension in the top-bottom direction in the figures) in the axial direction of the gas outlet 2, the stop structure 3 extends in the cavity 12 to a length not greater than half of the first dimension, to facilitate the gas to flow through the ventilation channel successfully.

The nasal plug for a nasal oxygen catheter in the present disclosure has a simple structure, is easy to manufacture, has a low production cost, can effectively inhibit the condensed water in the cavity 12 of the nasal plug from entering the respiratory tract of the patient through the gas outlets 2, thereby optimizes the user experience and improves the competitiveness of the product.

It needs to be noted that the nasal plug described above may be used in any type of nasal oxygen catheters. Accordingly, in a second aspect, the present disclosure provides a nasal oxygen catheter, which comprises a ventilation pipeline and the above-mentioned nasal plug for a nasal oxygen catheter.

In a third aspect, the present disclosure provides a ventilation therapy device, which comprises the above-mentioned nasal oxygen catheter.

The ventilation therapy device may comprise a main unit that serves as a gas source and a ventilation pipeline connected to the main unit, wherein the ventilation pipeline may comprise the above-mentioned nasal oxygen catheter.

During use, the gas from the main unit enters the nasal plug through the ventilation pipeline, and then enters the nasal cavity of the user through the nasal plug.

In the present disclosure, the ventilation therapy device may be a ventilator or an oxygen therapy device, such as a high-flux oxygen therapy device or a high-flux humidifying therapy device.

In the description of the present disclosure, the expressions of reference terms "an embodiment", "some embodiments", "an example", "specific example", or "some examples" mean that the specific features, structures, materials or characteristics described in those embodiments or examples are included in at least one embodiment or example of the present disclosure. In this document, the exemplary expression of the above terms may not necessarily refer to the same embodiment or example. Moreover, the specific features, structures, materials, or characteristics described can be combined appropriately in any one or more embodiments or example. Furthermore, those skilled in the art may combine or assemble different embodiments or examples and features in different embodiments or examples described herein, provided that there is no conflict among them.

## Claims

1. A nasal plug for a nasal oxygen catheter, comprising a body (1), which comprises a casing (11) and a cavity (12) positioned in the casing (11), and is provided with at least one gas outlet (2), wherein the nasal plug further comprises a stop structure (3), **characterized in that** the stop structure (3) surrounds the at least one gas outlet (2) and extends from an inner wall of the casing (11) toward the cavity (12), so that condensed water on the inner wall of the cavity has to flow over the stop structure (3) before it can be discharged from the gas outlet (2).

2. The nasal plug of claim 1, wherein a reinforcing structure is formed on the stop structure (3).

3. The nasal plug of claim 2, wherein the reinforcing structure comprises reinforcing ribs (31).

4. The nasal plug of claim 2, wherein the reinforcing structure comprises a flanged edge (32), which is folded and extends towards the outside of the gas outlet (2).

5. The nasal plug of any of claims 1 - 4, wherein the stop structure (3) comprises a first end connected to the casing (11) and a second end extending into the cavity (12), and an inner diameter of the second end is greater than that of the gas outlet (2).

6. The nasal plug of claim 5, wherein the inner diameter of the second end is greater than that of the first end.

7. The nasal plug of claim 5 or 6, wherein the inner diameter of the first end is greater than that of the gas outlet (2).

8. The nasal plug of claim 5, wherein one part of the first end extends from an edge of the gas outlet (2), and another part of the first end is arranged spaced apart from the edge of the gas outlet (2).

9. The nasal plug of any of claims 1 - 8, wherein the stop structure (3) is a stop ring.

10. The nasal plug of any of claims 1 - 9, wherein the nasal plug comprises at least two gas outlets (2), and the stop structure (3) surrounds all the gas outlets (2) on the nasal plug.

11. The nasal plug of any of claims 1 - 10, wherein one end of the cavity (12) is formed as a gas inlet end, and the other end is formed as a closed end, and a connecting structure for connecting with a ventilation pipeline is formed on a side of the casing (11) corresponding to the gas inlet end.

12. A nasal oxygen catheter, comprising a ventilation pipeline and the nasal plug of any of claims 1 - 11.

13. A ventilation therapy device, comprising the nasal oxygen catheter of claim 12.

## Patentansprüche

1. Nasenstecker für einen nasalen Sauerstoffkatheter, umfassend einen Körper (1), der ein Gehäuse (11) und einen in dem Gehäuse (11) angeordneten Hohlraum (12) umfasst und mit mindestens einem Gasauslass (2) versehen ist, wobei der Nasenstecker ferner eine Anschlagstruktur (3) umfasst, **dadurch gekennzeichnet, dass** die Anschlagstruktur (3) den mindestens einen Gasauslass (2) umgibt und sich von einer Innenwand des Gehäuses (11) in Richtung des Hohlraums (12) erstreckt, so dass Kondenswasser an der Innenwand des Hohlraums über die Anschlagstruktur (3) fließen muss, bevor es aus dem Gasauslass (2) abgeführt werden kann.

2. Nasenstecker nach Anspruch 1, wobei eine Verstärkungsstruktur auf der Anschlagstruktur (3) ausgebildet ist.

3. Nasenstecker nach Anspruch 2, wobei die Verstärkungsstruktur Verstärkungsrippen (31) umfasst.

4. Nasenstecker nach Anspruch 2, wobei die Verstärkungsstruktur eine Flanschkante (32) umfasst, die gefaltet ist und sich zur Außenseite des Gasauslasses (2) erstreckt.

5. Nasenstecker nach einem der Ansprüche 1 bis 4, wobei die Anschlagstruktur (3) ein erstes Ende, das mit dem Gehäuse (11) verbunden ist, und ein zweites Ende, das sich in den Hohlraum (12) erstreckt, umfasst und ein Innendurchmesser des zweiten Endes größer ist als der des Gasauslasses (2).

6. Nasenstecker nach Anspruch 5, wobei der Innendurchmesser des zweiten Endes größer ist als der des ersten Endes.

7. Nasenstecker Nasestöpsel nach Anspruch 5 oder 6, wobei der Innendurchmesser des ersten Endes größer ist als der des Gasauslasses (2).

8. Nasenstecker nach Anspruch 5, wobei ein Teil des ersten Endes von einer Kante des Gasauslasses (2) ausgeht und ein anderer Teil des ersten Endes von der Kante des Gasauslasses (2) beabstandet angeordnet ist.

9. Nasenstecker nach einem der Ansprüche 1 bis 8, wobei die Anschlagstruktur (3) ein Anschlagring ist.

10. Nasenstecker nach einem der Ansprüche 1 bis 9, wobei der Nasenstecker mindestens zwei Gasauslässe (2) umfasst und die Anschlagstruktur (3) alle Gasauslässe (2) auf dem Nasenstecker umgibt.

11. Nasenstecker nach einem der Ansprüche 1 bis 10, wobei ein Ende des Hohlraums (12) als Gaseinlassende und das andere Ende als geschlossenes Ende ausgebildet ist und eine Verbindungsstruktur zum Verbinden mit einer Beatmungsleitung an einer Seite des Gehäuses (11) ausgebildet ist, die dem Gaseinlassende entspricht.

12. Nasaler Sauerstoffkatheter, umfassend eine Beatmungsleitung und den Nasenstecker nach einem der Ansprüche 1 bis 11.

13. Beatmungstherapiegerät, umfassend den nasalen Sauerstoffkatheter nach Anspruch 12.

## Revendications

1. Bouchon nasal pour un cathéter nasal à oxygène, comprenant un corps (1), qui comprend un boîtier (11) et une cavité (12) positionnés dans le boîtier (11), et qui est pourvu d'au moins un orifice de sortie de gaz (2), dans lequel le bouchon nasal comprend en outre une structure d'arrêt (3), **caractérisé en ce que** la structure d'arrêt (3) entoure le au moins un orifice de sortie de gaz (2) et s'étend depuis une paroi interne du boîtier (11) vers la cavité (12), de sorte que l'eau condensée sur la paroi interne de la cavité doit s'écouler sur la structure d'arrêt (3) avant qu'elle puisse être évacuée de l'orifice de sortie de gaz (2).

2. Bouchon nasal selon la revendication 1, dans lequel une structure de renforcement est formée sur la structure d'arrêt (3).

3. Bouchon nasal selon la revendication 2, dans lequel la structure de renforcement comprend des nervures de renforcement (31).

4. Bouchon nasal selon la revendication 2, dans lequel la structure de renforcement comprend un bord à collerette (32), qui est plié et qui s'étend vers l'extérieur de l'orifice de sortie de gaz (2).

5. Bouchon nasal selon l'une quelconque des revendications 1 à 4, dans lequel la structure d'arrêt (3) comprend une première extrémité raccordée au boîtier (11) et une seconde extrémité s'étendant dans la cavité (12), et un diamètre interne de la seconde extrémité est supérieur à celui de l'orifice de sortie de gaz (2).

6. Bouchon nasal selon la revendication 5, dans lequel le diamètre interne de la seconde extrémité est supérieur à celui de la première extrémité.

7. Bouchon nasal selon la revendication 5 ou 6, dans lequel le diamètre interne de la première extrémité est supérieur à celui de l'orifice de sortie de gaz (2).

8. Bouchon nasal selon la revendication 5, dans lequel une partie de la première extrémité s'étend depuis un bord de l'orifice de sortie de gaz (2), et une autre partie de la première extrémité est agencée espacée à l'écart du bord de l'orifice de sortie de gaz (2).

9. Bouchon nasal selon l'une quelconque des revendications 1 à 8, dans lequel la structure d'arrêt (3) est une bague d'arrêt.

10. Bouchon nasal selon l'une quelconque des revendications 1 à 9, dans lequel le bouchon nasal comprend au moins deux orifices de sortie de gaz (2), et la structure d'arrêt (3) entoure tous les orifices de sortie de gaz (2) sur le bouchon nasal.

11. Bouchon nasal selon l'une quelconque des revendications 1 à 10, dans lequel une extrémité de la cavité (12) est formée sous la forme d'une extrémité d'entrée de gaz, et l'autre extrémité est formée sous la forme d'une extrémité fermée, et une structure de raccordement pour le raccordement à un pipeline de ventilation est formée sur un côté du boîtier (11) correspondant à l'extrémité d'entrée de gaz.

12. Cathéter nasal à oxygène, comprenant un pipeline de ventilation et le bouchon nasal selon l'une quelconque des revendications 1 à 11.

13. Dispositif de thérapie par ventilation, comprenant le cathéter nasal à oxygène selon la revendication 12.
